# EUROPEAN PATENT SPECIFICATION

(11) **EP 2 095 797 B1**
(45) Date of publication and mention of the grant of the patent: **29.02.2012**
(21) Application number: 09250585.8
(22) Date of filing: 02.03.2009
(51) Int. Cl.: A61F 5/00

(54) **Devices for fixing antenna orientation in a restriction system**
Vorrichtungen zur Fixierung der Antennenausrichtung in einem Einschränkungssystem
Dispositifs de fixation d'orientation d'antenne dans un système de restriction

(30) Priority: 29.02.2008 US 40266
(43) Date of publication of application: 02.09.2009
(73) Proprietor: Ethicon Endo-Surgery, Inc., Cincinnati, OH 45242 (US)
(72) Inventor: Dlugos, Jr., Daniel F., Middletown, OH 45044 (US); Ortiz, Mark S., Milford, OH 45150 (US); Marcotte, Amy L., Mason, OH 45040 (US); Plescia, David N., Cincinnati, OH 45227 (US)
(74) Representative: Tunstall, Christopher Stephen

(56) References cited:
- EP-A- 1 736 123
- WO-A-99/38438
- WO-A-2006/040647
- WO-A-2007/140430
- US-A1- 2006 173 238
- US-A1- 2007 156 013

## Description

### FIELD

The present application relates to methods and devices for fixing antenna orientation in a restriction system.

### BACKGROUND

Obesity is a growing global concern, as the number of individuals classified as overweight, obese, or morbidly obese continues to increase every year. Obesity is associated with several co-morbidities, including hypertension, type II diabetes, and sleep apnea. Morbid obesity, defined as when a person is 100 pounds or more over ideal body weight or having a body mass index (BMI) of 40 or greater, poses the greatest risks for severe health problems. Accordingly, a great deal of attention is being focused on treating patients with this condition. One method of treating morbid obesity is the placement of a restriction device, such as an elongated band, around the upper portion of the stomach. Gastric bands are typically comprised of a fluid-filled elastomeric balloon with fixed endpoints that encircles the stomach just inferior to the esophageal-gastric junction. This forms a small gastric pouch above the band and a reduced stoma opening inferior to the gastro-esophageal junction. When fluid is infused into the balloon, the band expands against the stomach creating further food intake restriction or a smaller stoma opening in the stomach. To decrease this restriction level, fluid is removed from the band. The effect of the band is to reduce the available stomach volume and thus the amount of food that can be consumed before becoming "full."

Food restriction devices have also comprised mechanically adjusted bands that similarly encircle the upper portion of the stomach. These bands include any number of resilient materials or gearing devices, as well as drive members, for adjusting the bands. Additionally, gastric bands have been developed that include both hydraulic and mechanical drive elements. An example of such an adjustable gastric band is disclosed in U.S. Pat. No. 6,067,991, entitled "Mechanical Food Intake Restriction Device" which issued on May 30, 2000.
Another method for limiting the available food volume in the stomach cavity is implanting an inflatable elastomeric balloon within the stomach cavity itself. The balloon is filled with a fluid to expand against the stomach walls and, thereby, decrease the available food volume within the stomach.

With each of the above-described food limitation devices, safe, effective treatment requires that the device be regularly monitored and adjusted to vary the degree of affect on food intake. With banding devices, the gastric pouch above the band may substantially increase in size following the initial implantation. Accordingly, the stoma opening in the stomach must initially be made large enough to enable the patient to receive adequate nutrition while the stomach adapts to the banding device. As the patient's body adapts to the implant, the band may be adjusted to vary the stoma size. In addition, it is desirable to vary the stoma size in order to accommodate changes in the patient's body or treatment regime, or in a more urgent case, to relieve an obstruction or severe esophageal dysmotility or dilatation. Traditionally, adjusting a hydraulic gastric band required a scheduled clinician visit during which a Huber (non-coring) needle and syringe were used to penetrate the patient's skin and add or remove fluid from the balloon. More recently, devices have been developed which enable non-invasive adjustments of the band. An external programmer communicates with the implant using telemetry to control the stoma diameter of the band. During a scheduled visit, a physician places a hand-held portion of the programmer near the implant and transmits power and command signals to the implant. The implant in turn adjusts the stoma diameter of the band and transmits a response command to the programmer. WO2007140430, WO9938438, US2007156013, EP1736123, US2006173238, and WO2006040647 each provide a system comprising a gastric constriction device in communication with an external device via an internal antenna.

One problem that can arise is giving stability to various housings in a restriction system, such as an antenna housing for communicating with an external device. Specifically, it can be difficult to provide orientational stability to an antenna housing once it is implanted as the tissue underneath the skin does not provide a flat surface for mounting and the housing may shift locations as the patient loses or gains weight, or even during movement by the patient. As a result, it can be difficult to align an external device with the antenna housing to enable wireless communication.

Accordingly, there remains a need for improved methods and devices for substantially fixing the orientation of an antenna housing implanted in tissue.

### SUMMARY

The present invention provides a restriction system as defined in appended claim 1. Preferred embodiments are defined in the appended subclaims. Various devices are provided for substantially fixing the orientation of a housing, such as an antenna housing, within tissue. In one embodiment, a restriction system is provided having a first housing with a reservoir formed therein for receiving fluid. The first housing can be configured to be anchored to tissue. The system can also include a second housing spaced apart from and in fluid communication with the first housing. The second housing can have an antenna therein configured to wirelessly communicate with an external device. The system can also include a restriction device in fluid communication with the first and second housings and adapted to form a restriction in a pathway, and a constraining element coupled to the first and second housings and configured to limit rotational movement of the first and second housings relative to one another. In an exemplary embodiment, the constraining element is configured to substantially prevent rotation of the first and second housings along an axis extending between the first and second housings.

The constraining element can have various configurations. In one embodiment, the constraining element can be substantially rigid in a first plane of motion and flexible in a second plane of motion that differs from the first plane of motion. For example, the constraining element can be a sheath disposed around at least a portion of the first and second housings. In one embodiment, the sheath can be sealed with a hermetic coating. The system can also include a connector, such as a catheter, extending through the constraining element between the first and second housings. The connector can be configured to allow fluid flow therethrough between the first and second housings. Alternatively, the constraining element can include a lumen extending therethrough and configured to allow fluid flow between the first and second housings. The constraining element can also include other features, such as an outer layer formed from a compliant material, such as keratin and silicone.

In another embodiment, a restriction system is provided having a fill port with a needle-penetrable septum and a reservoir formed therein and configured to receive fluid. An antenna housing can be coupled to the fill port and it can have an antenna therein configured to wirelessly communicate with an external device. The system can also include a constraining element extending between the fill port and the antenna housing. The constraining element can be substantially rigid in a first plane of motion and flexible in a second plane of motion that differs from the first plane of motion. For example, the constraining element can prevent rotation between the antenna housing and the fill port. The constraining element can have various configurations, as discussed above.

### BRIEF DESCRIPTION OF THE DRAWINGS

The invention will be more fully understood from the following detailed description taken in conjunction with the accompanying drawings, in which:

FIG. 1A is a schematic diagram of one embodiment of a food intake restriction system;

FIG. 1B is perspective view of an implantable portion of the food intake restriction system of FIG. 1A;

FIG. 2A is a perspective view of a restriction device of the restriction system of FIG. 1A;

FIG. 2B is a perspective view of the restriction device of FIG. 2A applied about the gastro-esophageal junction of a stomach;

FIG. 3 is a perspective view of an injection port housing of FIG. 1A;

FIG. 4 is a perspective view of the implantable portion of the food intake restriction system of FIG. 1A showing a cross-sectional view of an antenna housing;

FIG. 5 is a perspective, partially transparent view of the antenna housing of FIG. 4;

FIG. 6 is a perspective view of one embodiment of a constraining element disposed around at least a portion of the injection port housing and antenna housing of FIGS. 1B and 4;

FIG. 7 is a perspective view of the constraining element of FIG. 6; and

FIG. 8 is a perspective cross-sectional view of the constraining element of FIG. 6 showing a lumen extending therethrough.

### DETAILED DESCRIPTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims.

Various exemplary methods and devices are provided for limiting movement of an antenna housing associated with a restriction system to allow for alignment and communication with an external device. In certain exemplary embodiments, the antenna housing is constrained relative to another housing, such as an injection port 30, that is anchored to tissue. In particular, a constraining element can be coupled to the housings and it can limit or substantially prevent movement of the housings relative to one another, preferably in at least one plane of motion. Since housings tend to shift once implanted in tissue, the ability to control movement between the housings can be particularly advantageous to allow for effective wireless communication with an external device.

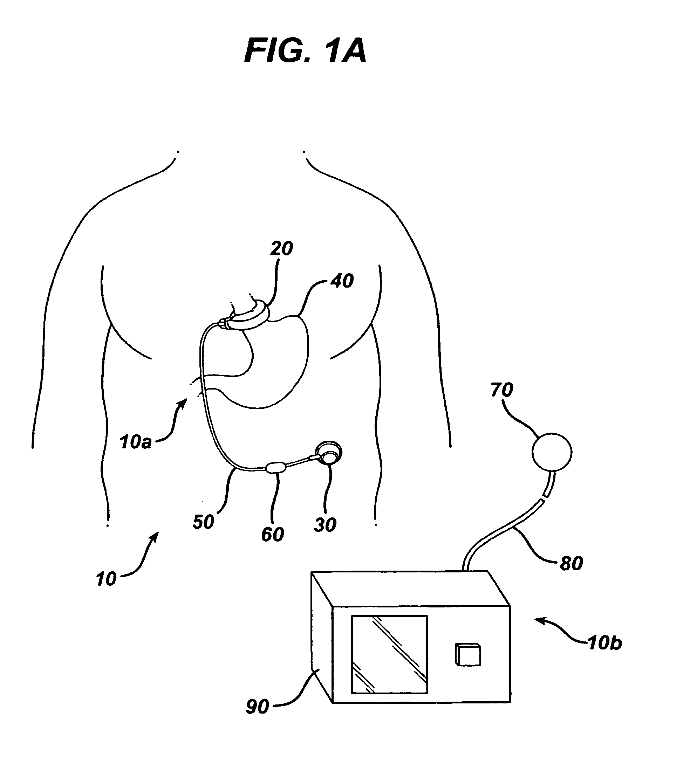

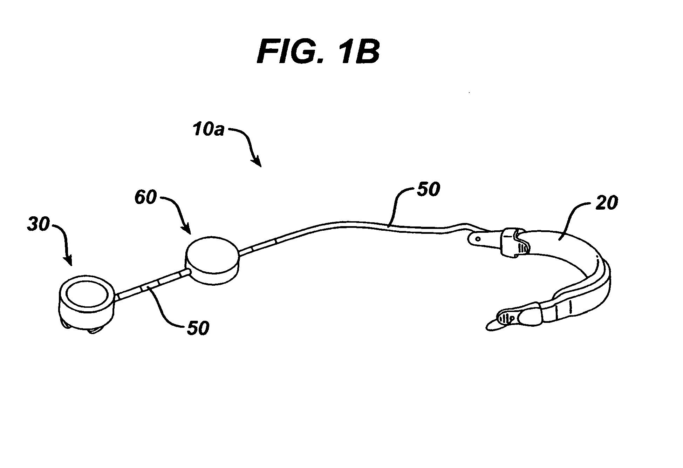

While the present invention can be used with a variety of restriction systems known in the art, FIG. 1 illustrates one exemplary embodiment of a food intake restriction system 10. As shown, the system 10 generally includes an implantable portion 10a having an adjustable gastric band 20 that is configured to be positioned around the upper portion of a patient's stomach 40, and an injection port 30 that is fluidly coupled to the adjustable gastric band 20, e.g., via a catheter 50. The injection port 30 is adapted to allow fluid to be introduced into and removed from the gastric band 20 to thereby adjust the size of the band, and thus the pressure applied to the stomach. The injection port 30 can thus be implanted at a location within the body that is accessible through the tissue. Typically, injection ports are positioned in the lateral subcostal region of the patient's abdomen under the skin and layers of fatty tissue. Surgeons also typically implant injection ports on the sternum of the patient. The implantable portion 10a can also include an antenna housing 60 configured to communicate wirelessly with an external device.

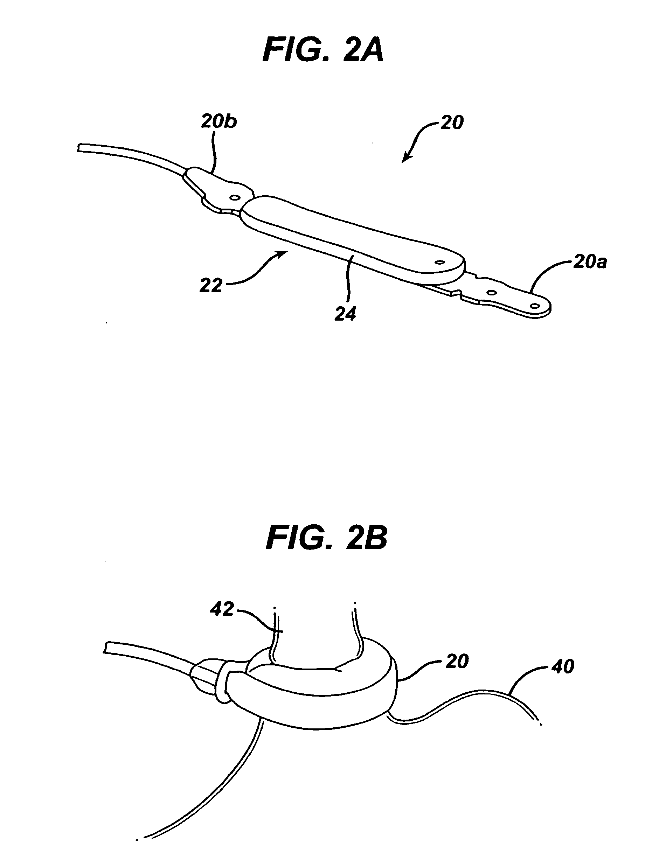

FIG. 2A shows the gastric band 20 in more detail. While the gastric band 20 can have a variety of configurations, and various gastric bands currently known in the art can be used with the present invention, in the illustrated embodiment the gastric band 20 has a generally elongated shape with a support structure 22 having first and second opposite ends 20a, 20b that can be secured to each other. Various mating techniques can be used to secure the ends 20a, 20b to one another. In the illustrated embodiment, the ends 20a, 20b are in the form of straps that mate together, with one laying on top of the other. The gastric band 20 can also include a variable volume member, such as an inflatable balloon 24, that is disposed or formed on one side of the support structure 22, and that is configured to be positioned adjacent to tissue. The balloon 24 can expand or contract against the outer wall of the stomach to form an adjustable stoma for controllably restricting food intake into the stomach. A person skilled in the art will appreciate that the gastric band can have a variety of other configurations, moreover the various methods and devices disclosed herein have equal applicability to other types of implantable bands. For example, bands are used for the treatment of fecal incontinence, as described in U.S. Pat. No. 6,461,292. Bands can also be used to treat urinary incontinence, as described in U.S. Patent Application 2003/0105385.
Bands can also be used to treat heartburn and/or acid reflux, as disclosed in U.S. Pat. No. 6,470,892. Bands can also be used to treat impotence, as described in U.S. Patent Application 2003/0114729.

FIG. 2B shows the adjustable gastric band 20 applied about the gastro-esophageal junction of a patient. As shown, the band 20 at least substantially encloses the upper portion of the stomach 40 near the junction with the esophagus 42. After the band 20 is implanted, preferably in the deflated configuration wherein the band 20 contains little or no fluid, the band 20 can be inflated, e.g., using saline, to decrease the size of the stoma opening. A person skilled in the art will appreciate that various techniques, including mechanical and electrical techniques, can be used to adjust the band.

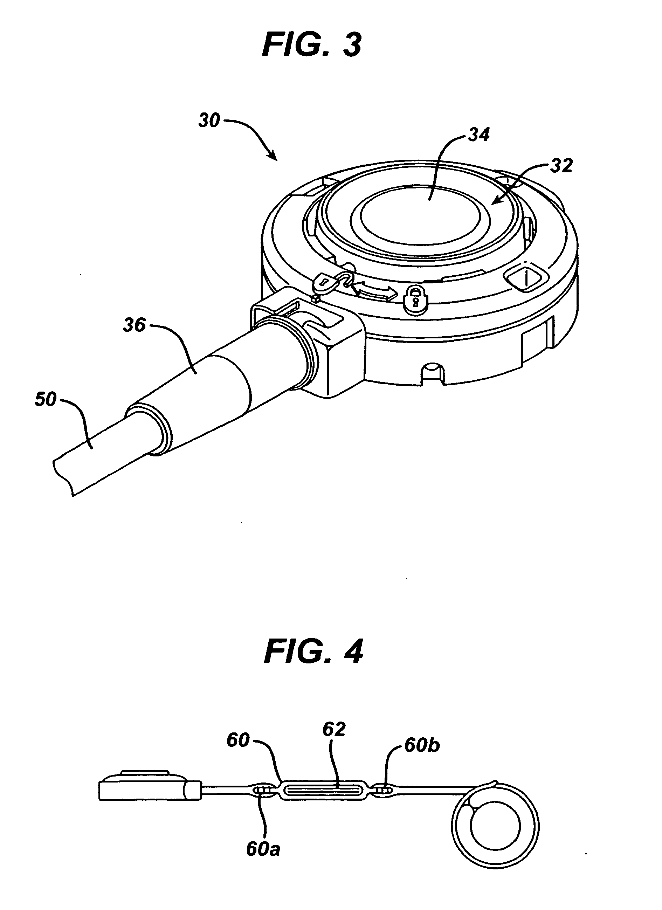

The fluid injection port 30 can also have a variety of configurations. In the embodiment shown in FIG. 3, the injection port 30 has a generally cylindrical housing with a distal or bottom surface and a perimeter wall extending proximally from the bottom surface and defining a proximal opening 32. The proximal opening 32 can include a needle-penetrable septum 34 extending there across and providing access to a fluid reservoir (not shown in FIG. 3) formed within the housing. The septum is preferably placed in a proximal enough position such that the depth of the reservoir is sufficient enough to expose the open tip of a needle, such as a Huber needle, so that fluid transfer can take place. The septum 34 is preferably arranged so that it will self seal after being punctured by a needle and the needle is withdrawn. As further shown in FIG. 3, the port can further include a catheter tube connection member 36 that is in fluid communication with the reservoir and that is configured to couple to a catheter (e.g., the catheter 50). A person skilled in the art will appreciate that the housing 30 can be made from any number of materials, including stainless steel, titanium, or polymeric materials, and the septum can likewise be made from any number of materials, including silicone. The port 30 can also be configured to be anchored to tissue. For example, the port 30 can include one or more suture-receiving members adapted to receive a suture for anchoring the port 30 to tissue, and/or the port 30 can include one or more anchors or hooks adapted to be deployed into tissue. A person skilled in the art will appreciate that any technique can be used to anchor the port 30 in tissue. Various techniques for anchoring an injection port to tissue are disclosed in more detail in commonly-owned U.S. Publication No. 2007/0208313 entitled "Method of Implanting a Fluid Injection Port," filed on May 7, 2007, and U.S. Publication No. 2007/0010790 entitled "Injection Port," filed June 24, 2005.

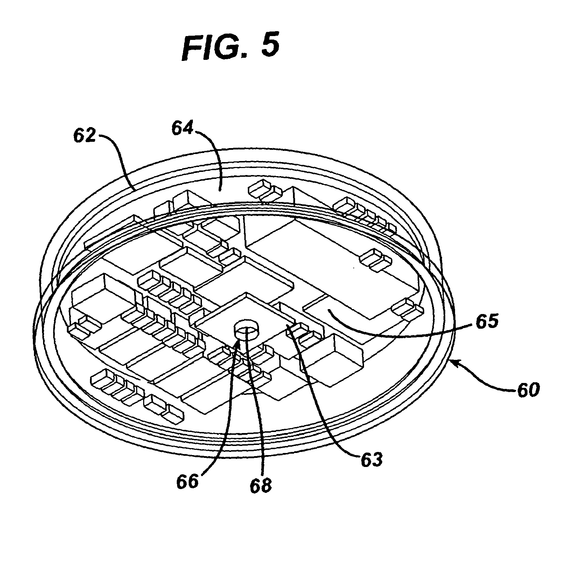

As indicated above and as shown in FIG. 4, the system can also include an antenna housing 60 having an antenna 62 therein that is configured to communicate wirelessly with an external device to allow power and/or data to be transferred between the antenna and the external device. The antenna 62 can be, for example, a TET/telemetry coil for inductively coupling with a TET/telemertry coil in an external device (e.g., a device external to the patient's body). The antenna 62 can be coupled to various components disposed within the antenna housing 60 or elsewhere within the system 10. For example, in one embodiment the antenna 62 can include or be coupled to a pressure measuring device that is in communication with the closed fluid circuit and that is configured to measure a fluid pressure that corresponds to the amount of restriction applied by the adjustable gastric band to the patient's stomach. Measuring the fluid pressure enables a physician to evaluate the restriction created by a band adjustment. In an exemplary embodiment, the pressure measuring device can be in the form of a pressure sensor 63, which can be unitary with the antenna 62 or a separate component, that can be disposed within the housing 60. The pressure measuring device can, however, be disposed anywhere within the closed hydraulic circuit of the implantable portion, and various exemplary locations and configurations are disclosed in more detail in commonly-owned U.S. Publication No. 2006/0211913 entitled "Non-Invasive Pressure Measurement In a Fluid Adjustable Restrictive Device, filed on March 7, 2006. Optionally, the pressure sensing system can further include a temperature sensor (not shown). The sensing system can also be configured to measure a variety of other parameters, for example, pulse count and pulse width. In general, as shown in FIG. 4, the illustrated housing 60 includes an inlet 60a and an outlet 60b that are in fluid communication with the fluid in the system. The sensor can be disposed within the housing 60 and it can be configured to respond to fluid pressure changes within the hydraulic circuit and convert the pressure changes into a usable form of data. As shown in more detail in FIG. 5, the pressure sensing system can also include a motherboard 64 that can serve as at least a portion of a hermetic container to prevent fluid from contacting any elements disposed within the housing 60, except as discussed for the sensor. The housing 60 can be made from any biocompatible material appropriate for use in a body, such as a polymer, silicone, ceramic, glass, biocompatible metal, and other similar types of material. Furthermore, the housing 60 can be made from any one or more of transparent (as shown in FIG. 5), opaque, semi-opaque, and radio-opaque material. The motherboard 64 including, among other elements, a microcontroller 65 (e.g., a processor), can also be disposed within the housing 60 to help process and communicate pressure measurements gathered by the sensor, and also possibly other data related to the band 20. As further discussed below, the motherboard 64 can also include a transcutaneous energy transfer (TET)/telemetry coil and a capacitor. Optionally, a temperature sensor can be integrated into the motherboard 64. The microcontroller 65, the TET/telemetry coil and/or antenna, the capacitor, and/or the temperature sensor can be in communication via the motherboard 64 or via any other suitable component(s). As indicated above, the TET/telemetry coil and/or antenna and capacitor can collectively form a tuned tank circuit for receiving power from the external portion 10b and transmitting pressure measurements to an external device, e.g., the reading device 70. The microcontroller and capacitor can be in communication via the motherboard or via any other suitable component(s). The antenna 62 disposed in the housing 60 can have a variety of configurations, but in the illustrated embodiment is in the form of a planar coil on the motherboard 64. The antenna 62 can be configured to emit field lines directed along an axis extending between superior and inferior surfaces of the housing 60. This can allow for optimal communication between the antenna 62 and an external device as it is preferable for the antenna 62 to be maintained in a position substantially parallel to a tissue surface.

Various pressure sensors known in the art can be used, such as a wireless pressure sensor provided by CardioMEMS, Inc. of Atlanta, Georgia, though a suitable MEMS pressure sensor may be obtained from any other source, including but not limited to Integrated Sensing Systems (ISSYS), and Remon Medical. One exemplary MEMS pressure sensor is described in U.S. Patent No. 6,855,115. It will also be appreciated that suitable pressure sensors may includes, but are not limited to, capacitive, piezoresistive, silicon strain gauge, or ultrasonic (acoustic) pressure sensors, as well as various other devices capable of measuring pressure.

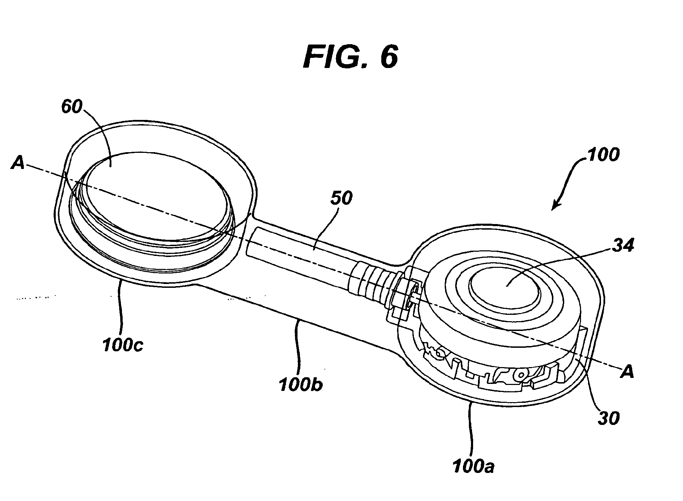

As discussed above, the system can include a constraining element coupled between first and second housings and configured to limit movement between first and second housings. This will allow the port 30, for example, to be anchored to tissue and the antenna housing 60 to be maintained in a substantially fixed orientation relative to the port 30. As a result, the antenna housing 60 can be maintained in a position substantially parallel to a tissue surface, thus allowing optimal communication between the antenna 62 and an external device. In an exemplary embodiment, the constraining element can be in the form of a sheath 100 extending between and optionally disposed around at least a portion of the injection port 30 and the antenna housing 60. The sheath 100 can be constructed to limit movement, e.g., rotational/torsional movement, between the housings preferably about an axis extending therebetween. Movement can be completely prevented, or merely limited in one or more directions or planes of motion. In an exemplary embodiment, as shown in FIG. 6, the sheath 100 has a first portion 100a configured to fit around the injection port 30, a second portion 100b adapted to extend between the injection port 30 and the sensor housing 60, and a third portion 100c adapted to fit around the sensor housing 60, as will be discussed in more detail below.

The sheath 100 can be formed from a variety of materials, and it can be rigid or flexible, but in the preferred embodiment the sheath 100 is at least semi-rigid to limit movement between the injection port 30 and the sensor housing 60. For example, the sheath 100 can be formed from an elastomeric material. In addition, the sheath 100 can be formed from a hermetic or near-hermetic material as the sheath 100 can also be configured to form a seal around the injection port 30 and the sensor housing 60. This seal can be configured to substantially eliminate transport of materials both into and out of the sheath 100 in order to provide protection to the components housed within the sheath 100, including the components housed in the injection port 30 and the sensor housing 60. A hermetic seal can be achieved with a variety of hermetic materials, such as laser welded titanium. A person skilled in the art will appreciate that the sheath 100 can be formed from any material that has the ability to form a hermetic seal using any known technique for forming a seal, including AuSn brazing, anodic bonding, seam welding, or impulse welding. A near-hermetic seal can be achieved using a variety of near-hermetic materials to form the sheath 100, such as materials configured to slow the ingress of moisture through the sheath. A near-hermetic seal can also be achieved through the use of a coating formed around the sheath. A person skilled in the art will appreciate that a variety of materials can form a near-hermetic seal, including but not limited to silicones, metallized LCP, parylene-C, PDMS, and PEEK. Moreover, a person skilled in the art will appreciate that a variety of technologies can be used to form a coating around the sheath 100, including nanoreinforced moisture barrier coatings and self-aligned nano-particle engineered surfaces. In addition, the sheath 100 can also be formed from a keratin. This can be advantageous as keratin is less likely to react or be rejected by the body as it is a substance found in the body, it is less susceptible to humidity, it can be gamma sterilized, and can be injection molded to form the various components of the sheath 100. Keratin has also been shown to accelerate tissue healing as it can cooperate with the body's healing mechanisms. A person skilled in the art will appreciate, however, that the sheath 100 can be formed from any material that can be implanted in the body and that can provide limitation of movement between the housings as described above.

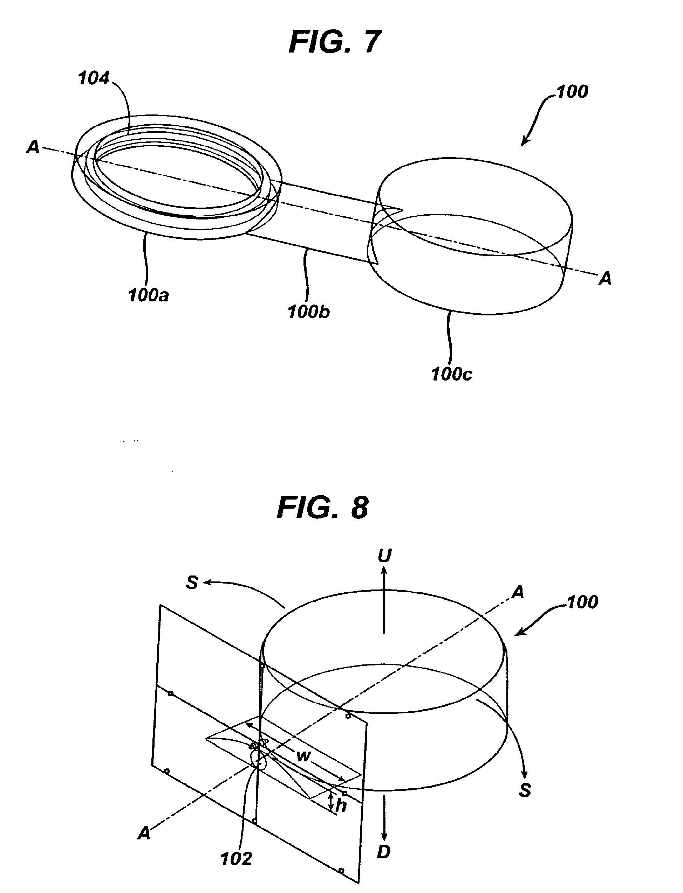

As discussed above, the first and third portions 100a, 100c of the sheath 100 can have any configuration that allows the sheath to fit aground and/or mate to the injection port 30 and the antenna housing 60, and the sheath 100 can be formed in a variety of ways. For example, the first portion 100a of the sheath 100 can be overmolded to encompass a portion of the injection port 30, but preferably not the entire injection port 30. Specifically, at least the septum 34 extending across the injection port 30 will not be encompassed by the sheath 100 as the septum 34 is configured to provide access to the fluid reservoir formed within the housing of the injection port 30. The sheath 100 can, however, be needle-penetrable to allow fluid to be introduced into an injection port 30 fully encapsulated by the sheath 100. In an exemplary embodiment illustrated in FIG. 7, the first portion 100a of the sheath 100 is formed around an engagement flange 104 formed on or matable to the injection port 30. The third portion 100c of the sheath 100 can likewise be overmolded to encapsulate a portion of or preferably the entire housing 60. A person skilled in the art will appreciate that as much or as little of the injection port 30 and/or the housing 60 can be encapsulated by the sheath 100, but preferably the sheath 100 is configured to provide enough of a seal to protect the components within the injection port 30 and the antenna housing 60 and provide enough stability to the injection port 30 and the housing 60 to limit movement therebetween. In other embodiments, the sheath 100 need not include the first and third portions 100a, 100c, but rather can merely extend between the port 30 and the housing 60 without encapsulating the port 30 and the housing 60.

The second portion 100b of the sheath 100 can also have a variety of configurations. In an exemplary embodiment, the second portion 100b includes a lumen 102 extending therethrough that is configured to allow for fluid flow between the injection port 30 and the antenna housing 60. In one exemplary embodiment, the lumen 102 can contain a catheter 50, as shown in FIG. 6, extending therethrough to allow for fluid flow through the catheter 50 between the injection port 30 and the antenna housing 60. This can be achieved either by forming the sheath 100 around the catheter 50, or the catheter 50 can be routed through the lumen 102 extending through the sheath 100. The catheter 50 can have a variety of configurations, but is preferably sized and shaped to fit within the lumen 102 of the sheath 100. The catheter 50 can have a length that allows the catheter 50 to extend a distance between the injection port 30 to the antenna housing 60 such that the port 30 and the housing 60 are spaced a distance apart. In another exemplary embodiment, the lumen 102 of the sheath 100 can be configured to allow fluid flow therethrough without the need for a catheter 50 within the lumen 102. The lumen 102 can have any size and shape that allows for fluid to flow between the injection port 30 and the sensor housing 60.

As previously indicated, the second portion 100b of the sheath 100 is also preferably adapted to limit movement between the port 30 and the housing 60. While various techniques can be used to limit movement, in an exemplary embodiment the second portion 100b can be configured to provide rigidity in any or all planes or axes of movement. In one exemplary embodiment, the second portion 100b of the sheath 100 is configured to limit movement about an axis A (FIG. 6) extending between the port 30 and the housing 60 to prevent rotational movement between the injection port 30 and the housing 60. A person skilled in the art will appreciate that the rigidity and/or shape of the second portion 100b of the sheath 100 can be chosen to achieve a desired amount of constraint on movement. For example, the rigidity and/or shape of the second portion 100b of the sheath 100 can be selected to allow the injection port 30 and the housing 60 to be easily introduced in the body, for example, by allowing bending motion into and out of a plane containing the sheath 100, the port 30, and the housing 60. However, the rigidity and/or shape of the sheath 100 can prevent torsional movement of the injection port 30 and the housing 60 about the axis A extending between the port 30 and the housing 60 to keep the antenna 62 within the antenna housing 60 in a specific orientation in relation to the port 30, and thus the skin surface. This can be advantageous as it allows for implantation of the antenna 62 in a specific and known position and orientation in relation to the port 30 and thus the skin surface to increase the ease with which the antenna 62 can communicate with an external device positioned adjacent the skin surface. In addition, the rigidity and/or shape of the second portion 100b of the sheath 100 can also be selected to allow the catheter 50 to be easily routed through the lumen 102 formed in the sheath 100 in an embodiment described above in which the sheath 100 is not formed around the catheter and the catheter needs to be inserted through the lumen 102. For example, if the injection port 30 is not placed in the same plane as the antenna housing 60, the rotational stability provided by the sheath 100 can increase the ease with which the catheter is positioned within the lumen 102 in the sheath 100.

In an exemplary embodiment, as shown, the second portion 100b of the sheath 100 can have a shape that allows for bending between the port 30 and the housing 60 but prevents rotation therebetween. As best shown in FIG. 8, the second portion 100b of the sheath 100 has a generally elongate substantially flat configuration that limits or prevents rotational movement between the injection port 30 and the antenna housing 60 along the axis A of the second portion 100b of the sheath 100, but yet allows bending to occur along the axis A. in particular, the second portion 100b of the sheath 100 has a width w that is greater than a height h. The shortness of the height h is chosen to allow the port 30 and the housing 60 to bend in a first direction (i.e., in an out of a plane extending through the port 30 and the housing 60) while the wider width w prevents bending in a second direction that is substantially perpendicular to the first direction. In other words, the port 30 and the housing 60 can move up and down out of the plane in the direction U, D indicated in FIG. 8, but are prevented from moving sideways within the plane in the direction S indicated in FIG. 8, in addition to being prevented from rotating relative to one another along the axis. This results in the limitation of rotation about the axis A extending between the port 30 and the housing 60. In other words, the sheath 100 can act as a spring to prevent twisting about the axis A of the second portion 100b of the sheath 100 (i.e., the axis A extending between the housings). The sheath 100 can also have a flat distal surface that facilitates positioning of the sheath 100 on a tissue surface during and after implantation, and a convex proximal surface that can be curved to accommodate the lumen 102 extending through the second portion 100b of the sheath 100, while still maintaining a low profile. The second portion 100b of the sheath can also extend between the port 30 and the housing 60 between distal portions of the port 30 and the housing 60 to allow the flat distal surface of the sheath 100 to be co-planar with the distal surfaces of the port 30 and the housing 60, and thus the sheath 100, the port 30, and the housing 60 can rest on a tissue surface in one plane, for example, to ease implantation and anchoring the port 30 to tissue. A person skilled in the art will appreciate, however, that the second portion 100b of the sheath 100 can have any configuration adapted to limit movement between the injection port 30 and the housing 60.

In use, the restriction system 10 shown in FIG. 1A can be implanted using techniques known in the art. For example, the gastric band 20 can be introduced into the patient's body and positioned around the stomach to restrict the pathway into the stomach, thus limiting food intake. The housing 60 and the port 30 can be implanted in tissue, preferably in the fascia, and they can be coupled to the band 20 to allow fluid communication therebetween. Preferably, the port 30 is anchored to a surface of the fascia, such that the port 30 is substantially parallel to the skin surface to allow access to the port 30. As a result of the position of the port 30, the antenna housing 60, which is spaced a distance apart from the port 30 and preferably positioned on the fascia, will be limited or prevented from moving due to the constraining member.

After implantation, it is necessary to be able to communicate with the restriction system, for example, to transmit power to the restriction system and/or communicate data to and from the restriction system. Since the sheath 100 limits or prevents movement of the housing 60 containing the antenna 62 relative to the injection port 30, an external device placed on the skin surface above the housing 60 will be aligned with and can thus communicate with the antenna 62. For example, the sheath 100 can prevent rotational movement between the port 30 and the housing 60 along an axis extending therebetween, while allowing for bending in a plane of motion substantially perpendicular to the axis extending between the port 30 and the housing 60. This prevention of rotational movement will allow the antenna 62 and the external device to be substantially parallel to one another as the sheath 100 prevents the antenna 62 from rotating away from the surface of the tissue.

The devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces, and subsequent reassembly. In particular, the device can be disassembled, and any number of the particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly.

Preferably, the invention described herein will be processed before surgery. First, a new or used instrument is obtained and if necessary cleaned. The instrument can then be sterilized. In one sterilization technique, the instrument is placed in a closed and sealed container, such as a plastic or TYVEK bag. The container and instrument are then placed in a field of radiation that can penetrate the container, such as gamma radiation, x-rays, or high-energy electrons. The radiation kills bacteria on the instrument and in the container. The sterilized instrument can then be stored in the sterile container. The sealed container keeps the instrument sterile until it is opened in the medical facility.

It is preferred that device is sterilized. This can be done by any number of ways known to those skilled in the art including beta or gamma radiation, ethylene oxide, steam.

One of ordinary skill in the art will appreciate further features and advantages of the invention based on the above-described embodiments. Accordingly, the invention is not to be limited by what has been particularly shown and described, except as indicated by the appended claims.

## Claims

1. A restriction system, comprising:
a first housing (30) having a reservoir formed therein for receiving fluid, the first housing being configured to be anchored to tissue;
a second housing (60) spaced apart from and in fluid communication with the first housing, the second housing having an antenna (62) therein configured to wirelessly communicate with an external device;
a restriction device (20) in fluid communication with the first and second housings and adapted to form a restriction in a pathway; and
**characterized by** a constraining element (100) coupled to the first and second housings and configured to limit rotational movement of the first and second housings relative to one another.

2. The system of claim 1, wherein the constraining element is configured to substantially prevent rotation of the first and second housings along an axis extending between the first and second housings.

3. The system of claim 1, wherein the constraining element is substantially rigid in a first plane of motion and flexible in a second plane of motion that differs from the first plane of motion.

4. The system of claim 1, wherein the constraining element comprises a sheath (100) disposed around at least a portion of the first and second housings.

5. The system of claim 4, wherein the sheath is sealed with a hermetic coating.

6. The system of claim 1, further comprising a connector (50) extending through the constraining element between the first and second housings, the connector configured to allow fluid flow therethrough between the first and second housings.

7. The system of claim 1, wherein the constraining element includes a lumen (102) extending therethrough and configured to allow fluid flow between the first and second housings.

8. The system of claim 1, wherein the constraining element includes an outer layer formed from a compliant material.

9. The system of claim 8, wherein the compliant material is selected from the group consisting of keratin and silicone.

10. The restriction system of any preceding claim, wherein the first housing has a needle-penetrable septum (34).

## Patentansprüche

1. Restriktionssystem, das Folgendes umfasst:
ein erstes Gehäuse (30) mit einem darin geformten Reservoir zur Aufnahme von Flüssigkeit, wobei das erste Gehäuse in Gewebe verankert werden kann;
ein zweites Gehäuse (60), das vom ersten Gehäuse im Abstand angeordnet ist und mit diesem in Flüssigkeitsverbindung steht, wobei das zweite Gehäuse eine Antenne (62) darin aufweist, die zur drahtlosen Kommunikation mit einer externen Vorrichtung konfiguriert ist;
eine Restriktionsvorrichtung (20) in Flüssigkeitsverbindung mit den ersten und zweiten Gehäusen zur Bildung einer Restriktion in einem Pfad;
und
**gekennzeichnet durch** ein Hemmelement (100), das mit den ersten und zweiten Gehäusen verbunden ist und eine Drehbewegung der ersten und zweiten Gehäuse relativ zueinander begrenzen kann.

2. System nach Anspruch 1, worin das Hemmelement eine Drehung der ersten und zweiten Gehäuse entlang einer Achse, die sich zwischen den ersten und zweiten Gehäusen erstreckt, im Wesentlichen verhindern kann.

3. System nach Anspruch 1, worin das Hemmelement in einer ersten Bewegungsebene im Wesentlichen starr und in einer zweiten Bewegungsebene, die sich von der ersten Bewegungsebene unterscheidet, flexibel ist.

4. System nach Anspruch 1, worin das Hemmelement eine Hülle (100) umfasst, die zumindest um einen Teil der ersten und zweiten Gehäuse angeordnet ist.

5. System nach Anspruch 4, worin die Hülle mit einer hermetischen Dichtung versiegelt ist.

6. System nach Anspruch 1, das ferner einen Konnektor (50) umfasst, der sich durch das Hemmelement zwischen den ersten und zweiten Gehäusen erstreckt, wobei der Konnektor eine Flüssigkeitsströmung durch ihn hindurch zwischen den ersten und zweiten Gehäusen gestattet.

7. System nach Anspruch 1, worin das Hemmelement ein Lumen (102) aufweist, das sich durch es hindurch erstreckt und eine Flüssigkeitsströmung zwischen den ersten und zweiten Gehäusen gestattet.

8. System nach Anspruch 1, worin das Hemmelement eine aus einem nachgiebigen Material geformte Außenschicht aufweist.

9. System nach Anspruch 8, worin das nachgiebige Material aus der aus Keratin und Silikon bestehenden Gruppe ausgewählt ist.

10. Restriktionssystem nach einem der vorhergehenden Ansprüche, worin das erste Gehäuse ein von einer Nadel penetrierbares Septum (34) aufweist.

## Revendications

1. Système de restriction, comportant :
un premier logement (30) muni d'un réservoir formé à l'intérieur de celui-ci pour recevoir un fluide, le premier logement étant configuré pour être ancré dans le tissu ;
un second logement (60) espacé par rapport au premier logement et en communication fluidique avec celui-ci, le second logement étant muni d'une antenne (62) située à l'intérieur configurée pour communiquer sans fil avec un dispositif extérieur ;
un dispositif (20) de restriction en communication fluidique avec les premier et second logements et conçu pour former une restriction dans une voie ; et
**caractérisé par** un élément (100) de contrainte accouplé aux premier et second logements et configuré pour limiter un mouvement de rotation des premier et second logements l'un par rapport à l'autre.

2. Système selon la revendication 1, dans lequel l'élément de contrainte est configuré pour empêcher sensiblement la rotation des premier et second logements le long d'un axe s'étendant entre les premier et second logements.

3. Système selon la revendication 1, dans lequel l'élément de contrainte est sensiblement rigide dans un premier plan de mouvement et souple dans un second plan de mouvement qui est différent du premier plan de mouvement.

4. Système selon la revendication 1, dans lequel l'élément de contrainte comporte une gaine (100) disposée autour d'au moins une partie des premier et second logements.

5. Système selon la revendication 4, dans lequel la gaine est scellée au moyen d'un revêtement hermétique.

6. Système selon la revendication 1, comportant en outre un connecter (50) traversant l'élément de contrainte entre les premier et second logements, le connecteur étant configuré pour permettre l'écoulement de fluide à travers celui-ci entre les premier et second logements.

7. Système selon la revendication 1, dans lequel l'élément de contrainte comprend une lumière (102) traversante configurée pour permettre l'écoulement de fluide entre les premier et second logements.

8. Système selon la revendication 1, dans lequel l'élément de contrainte comprend une couche externe formée d'un matériau élastique.

9. Système selon la revendication 8, dans lequel le matériau élastique est choisi dans le groupe constitué de kératine et de silicone.

10. Système de restriction selon l'une quelconque des revendications précédentes, dans lequel le premier logement est muni d'un septum (34) transperçable par une aiguille.
